(19) Europäisches Patentamt
European Patent Office
Office européen des brevets

(11) Numéro de publication: **0 210 156**
**B1**

(12) # FASCICULE DE BREVET EUROPÉEN

(45) Date de publication du fascicule du brevet: **25.01.89**

(51) Int. Cl.⁴: **C 07 D 307/80**, C 07 F 5/06

(21) Numéro de dépôt: **86870090.7**

(22) Date de dépôt: **23.06.86**

(54) **Complexes moléculaires formés d'un dérivé de benzofuranne et de chlorure d'aluminium, leur préparation et leur utilisation.**

(30) Priorité: **25.06.85 FR 8509669**

(43) Date de publication de la demande: **28.01.87 Bulletin 87/5**

(45) Mention de la délivrance du brevet: **25.01.89 Bulletin 89/4**

(84) Etats contractants désignés: **AT BE CH DE FR GB IT LI LU NL SE**

(56) Documents cité: **CHEMICAL ABSTRACTS, vol. 83, no. 14, 6 octobre 1975, page 779, résumé no. 125489m, Columbus, Ohio, US; K.B. STAROWIEYSKI et al.: "Complexes of carbonyl compounds with RnAIX3-n compounds. III. Stoichiometry of complexes of ketones with aluminum trichloride", & J. ORGANOMET. CHEM. 1975, 94(3), 361-6**

(73) Titulaire: **SANOFI, 40, Avenue George V, F-75008 Paris (FR)**

(72) Inventeur: **Grain, Claude, Ville "Les Olivettes" La Croix du Sud, F-04290 Volonne (FR)**
Inventeur: **Jammot, Fernand, Les Eygatières, F-04200 Sisteron (FR)**

(74) Mandataire: **Bressand, Georges, c/o CABINET LAVOIX 2 Place d'Estienne d'Orves, F-75441 Paris Cedex 09 (FR)**

**EP 0 210 156 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention se rapporte, d'une manière générale, à de nouveaux complexes moléculaires formés d'un dérivé de benzofuranne et de chlorure d'aluminium, à leur préparation ainsi qu'à leur utilisation.

Les complexes moléculaires de l'invention répondent à la formule générale:

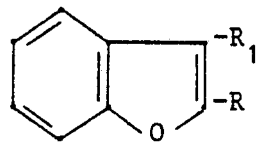

I

dans laquelle R représente un radical alkyle et $R_1$ représente l'un des radicaux:

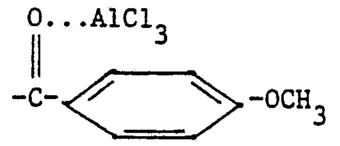

ou

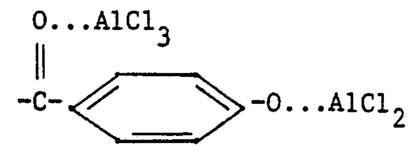

Par radical "alkyle", on désigne, dans le présent contexte, un radical hydrocarboné, linéaire ou ramifié ayant de 1 à 4 atomes de carbone plus spécialement un radical éthyle ou n-butyle.

Les complexes de formule I peuvent être largement utilisés comme intermédiaires de synthèse, notamment pour la synthèse finale de dérivés de benzofuranne décrits dans les brevets français Nos. 1 260 578 et 1 339 389. De tels dérivés sont notamment la benzarone ou éthyl-2 (hydroxy-4 benzoyl)-3 benzofuranne, la benziodarone ou éthyl-2(diiodo-3,5 hydroxy-4 benzoyl)-3 benzofuranne, la benzbromarone ou éthyl-2 (dibromo-3,5 hydroxy-4 benzoyl)-3 benzofuranne ou l'amiodarone ou n-butyl-2(diiodo-3,5 β-diéthylaminoéthoxy-4 benzoyl)-3 benzofuranne.

Ces composés se sont révélés particulièrement intéressants pour leurs applications thérapeutiques. Ainsi, la benzarone s'est montrée utile par son effet phlébotonique et freinateur des réactions inflammatoires capillaroveineuses, la benziodarone, par son action coronarodilatatrice et hypouricémiante, la benzbromarone par son effet hypouricémiant et l'amiodarone par ses propriétés antiangineuses et antiarythmiques cardiaques.

Un premier objet de l'invention se rapporte en conséquence aux complexes moléculaires de formule I en tant que produits industriels nouveaux utiles notamment comme intermédiaires par exemple pour la synthèse finale de la benzarone, la benziodarone, la benzbromarone et l'amiodarone.

Il est connu que les bases de Lewis telles que les cétones, les éthers, les amides peuvent former des complexes moléculaires avec des acides de Lewis tels que le chlorure de titane, de fer ou d'aluminium.

Des complexes plus ou moins stables, par exemple de type

FIG00/10

ont parfois été isolés et étudiés à l'état solide malgré leur instabilité et hygroscopicité importantes.

La composition de ces complexes est variable selon les conditions d'obtention et les rapports molaires base/acide.

Des exemples de tels complexes entre la benzophénone ou l'acétophénone d'une part et le chlorure d'aluminium d'autre part ont été décrits dans Helv. Chim. Acta, 1954, Vol. XXXVII, IV, 147, 1273 et 1958, Vol. XLI V, 146, 1333. Cependant, aucun complexe moléculaire dérivé de benzofuranne/acide de Lewis en particulier chlorure d'aluminium n'a été rapporté jusqu'à présent.

En outre, on sait que le chlorure d'aluminium anhydre est totalement insoluble dans les hydrocarbures aromatiques tels que le benzène ou le toluène.

Or, on a pu remarquer dans le cadre de la présente invention, que ce produit se solubilise à température inférieure ou égale à 40°C pratiquement instantanément dans des solutions toluéniques ou benzéniques d'alkyl-2 (méthoxy-4 benzoyl)-3 benzofuranne.

Cette "dissolution" s'accompagne d'une forte exothermicité et peut s'observer pour des rapports molaires chlorure d'aluminium/alkyl-2 (méthoxy-4 benzoyl)-3 benzofuranne allant jusqu'à 2,6.

On a alors émis l'hypothèse que cette dissolution du chlorure d'aluminium dans de telles solutions d'hydrocarbure aromatique pourrait provenir de la formation d'un ou de plusieurs complexes moléculaires entre le chlorure d'aluminium et l'alkyl-2 (méthoxy-4 benzoyl)-3 benzofuranne.

En conséquence, on a entrepris des études spectrales de manière à vérifier cette hypothèse et définir l'interaction supposée entre l'alkyl-2 (méthoxy-4 benzoyl)-3 benzofuranne et le chlorure d'aluminium.

A titre d'exemple, on a obtenu les résultats suivants avec le chlorure d'aluminium et le n-butyl-2 (méthoxy-4 benzoyl)-3 benzofuranne.

On a réalisé des solutions ternaires chlorure d'aluminium/n-butyl-2 (méthoxy-4 benzoyl)-3 benzofuranne/benzène deutéré et on y a fait varier, de 0 à 3, le rapport molaire:

$$r = \frac{\text{chlorure d'aluminium}}{\text{n-butyl-2 (méthoxy-4 benzoyl)-3 benzofuranne}}$$

Ainsi, on a pu mettre en évidence:

A. Par spectroscopie R.M.N. (résonnance magnétique nucléaire), l'absence de modification du groupement $-OCH_3$ entre les rapports molaires 0 et 1 ($\delta$ = 3,2 ppm) et une augmentation de déplacement chimique de + 0,35 ppm entre les rapports 1 et 3 qui peut correspondre à une interaction éther-$AlCl_3$.

B. Par spectroscopie I.R. (infra-rouge), l'absence pratiquement de modifications des absorptions caractéristiques des groupements C-O-C dans la zone 1200-1000 $cm^{-1}$, dans le cas présent le groupement C-O-C du benzofuranne. Dans le toluène également par spectroscopie I.R. on a observé, pour des rapports molaires variant de 0 à 1,5 et lors de l'ajout de chlorure d'aluminium, la disparition de la bande $\nu$ C = O "libre" à 1654 $cm^{-1}$ du n-butyl-2 (méthoxy-4 benzoyl)-3 benzofuranne au profit d'une bande située à 1545 $cm^{-1}$ (bande $\nu$ C = O "associée").

L'abaissement de la fréquence de 100 $cm^{-1}$ est l'indice d'une forte complexation. La disparition quasi totale de la bande libre pour le rapport molaire r = 1 montre que la totalité du n-butyl-2 (méthoxy-4 benzoyl)-3 benzofuranne est, à ce stade, engagé dans un complexe avec le chlorure d'aluminium et que ce dernier est probablement du type 1 : 1 sur le site cétone. Ce complexe moléculaire correspondrait donc à la formule:

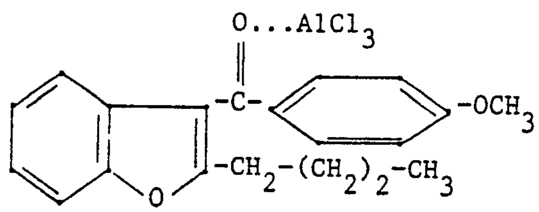

Le complexe 1 : 1 ainsi défini a pu être isolé à partir de solutions chlorométhyléniques.

Il se présente sous la forme d'un solide jaune mal cristallisé. Maintenu à l'abri de l'humidité et à basse température ($\leqslant$ 20°C), ce complexe apparaît stable. Il fond entre 25 et 40°C en se décomposant.

Le spectre I.R. du complexe moléculaire 1 : 1 en question, obtenu en opérant très rapidement pour éviter une hydrolyse (suspension dans une paraffine liquide) montre, par comparaison au spectre du n-butyl-2 (méthoxy-4 benzoyl)-3 benzofuranne en film, la disparition de la bande $\nu$ C = O "libre". La bande $\nu$ C = O "associée" apparaît large et mal définie entre 1500 et 1550 $cm^{-1}$.

Les spectres I.R. et R.M.N. du complexe 1 : 1 après dissolution dans le toluène ou le benzène montrent très exactement les perturbations spectrales observées lors de l'étude effectuée sur les solutions ternaires pour le rapport molaire r = 1 et dont les résultats sont rapportés précédemment.

Les résultats des études spectrales des solutions ternaires ont également laissé supposer la formation d'un complexe moléculaire chlorure d'aluminium/n-butyl-2 (méthoxy-4 benzoyl)-3 benzofuranne dans le rapport molaire 2 : 1. Cependant, le fait qu'il s'agisse d'un complexe 2 : 1 n'a pas été prouvé.

Par contre, on a également pu mettre en évidence que des solutions toluéniques contenant le chlorure d'aluminium et le n-butyl-2 (méthoxy-4 benzoyl)-3 benzofuranne dans le rapport r = 2 donnent naissance à un nouveau complexe par addition de ces solutions dans un hydrocarbure aromatique par exemple le benzène ou le toluène maintenu au reflux. Ce nouveau complexe correspondant à un complexe de déméthylation, répond à la formule:

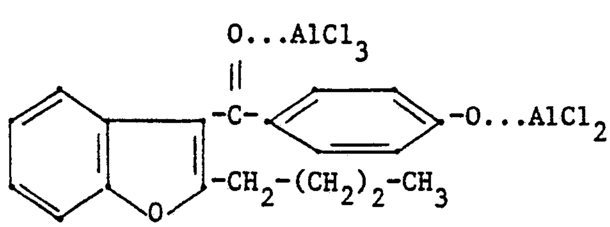

Il se présente sous forme d'une poudre jaunâtre finement divisée fondant au delà de 300°C avec décomposition.

En conclusion, on peut affirmer qu'il y a interaction du groupement cétonique de l'alkyl-2 (méthoxy-4 benzoyl)-3 benzofuranne avec le chlorure d'aluminium à basse température ($\leqslant$ 20°C) dans un hydrocarbure aromatique tel que le benzène ou le toluène et ce, pour des rapports molaires chlorure d'aluminium/alkyl-2 (méthoxy-4 benzoyl)-3 benzofuranne allant jusqu'à 2, qu'il n'y a aucune interaction avec le groupement C-O-C benzofurannique et qu'une interaction faible apparaît avec le groupement éther uniquement au-delà du rapport 1.

On peut donc supposer que le complexe hypothétique chlorure d'aluminium/alkyl-2 (méthoxy-4 benzoyl)-3 benzofuranne 2 : 1 qui se formerait à froid (température ⩽ 20°C) dans un hydrocarbure aromatique comme le benzène ou le toluène, se modifierait thermiquement avec migration d'une molécule de chlorure d'aluminium du groupement cétone vers le groupement éther de l'alkyl-2 (méthoxy-4 benzoyl)-3 benzofuranne pour permettre la déméthylation.

Un autre objet de l'invention se rapporte à un procédé de préparation des complexes moléculaires 1 : 1 de formule I dans laquelle $R_1$ représente le radical

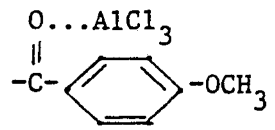

procédé selon lequel on fait réagir le chlorure d'aluminium et l'alkyl-2 (méthoxy-4 benzoyl)-3 benzofuranne dans le rapport 1 à 2 : 1, en milieu hydrocarbure aromatique tel que par exemple toluène ou benzène ou en milieu chloroalkane tel que dichlorométhane et à température inférieure ou égale à la température ambiante pour obtenir le complexe chlorure d'aluminium/alkyl-2 (méthoxy-4 benzoyl)-3 benzofuranne 1 : 1.

De même, l'invention se rapporte à un procédé de préparation des complexes moléculaires 2 : 1 de formule I dans laquelle $R_1$ représente le radical

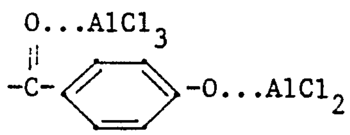

procédé selon lequel on porte au reflux une solution contenant le complexe 1 : 1 selon l'invention c'est-à-dire une solution formée au départ de 1 à 2 moles de chlorure d'aluminium par mole d'alkyl-2 (méthoxy-4 benzoyl)-3 benzofuranne et d'un hydrocarbure aromatique tel que le benzène ou le toluène comme solvant, pour obtenir le complexe chlorure d'aluminium/alkyl-2 (hydroxy-4 benzoyl)-3 benzofuranne 2 : 1.

Selon une mise en oeuvre préférée, on réalise à une température inférieure ou égale à la température ambiante, une solution de 1 à 2 moles de chlorure d'aluminium par mole d'alkyl-2 (méthoxy-4 benzoyl)-3 benzofuranne dans un hydrocarbure aromatique. On coule alors, dans un hydrocarbure aromatique porté au reflux, la solution ainsi obtenue qui contient le complexe chlorure d'aluminium/alkyl-2 (méthoxy-4 benzoyl)-3 benzofuranne 1 : 1 selon l'invention de manière à former le complexe chlorure d'aluminium/alkyl-2 (hydroxy-4 benzoyl)-3 benzofuranne 2 : 1 selon l'invention.

L'invention concerne également une solution contenant les complexes moléculaires de l'invention et convenant notamment comme milieu réactionnel pour provoquer la déméthylation du groupement éther d'un alkyl-2 (méthoxy-4 benzoyl)-3 benzofuranne, solution formée au départ de 1 à 2 moles de chlorure d'aluminium par mole d'alkyl-2 (méthoxy-4 benzoyl)-3 benzofuranne et d'un hydrocarbure aromatique tel que le benzène ou le toluène comme solvant.

Cette solution, à température inférieure ou égale à la température ambiante, contient le complexe 1 : 1 selon l'invention et à la température de reflux, le complexe 2 : 1 de l'invention.

Les alkyl-2 (méthoxy-4 benzoyl)-3 benzofurannes de départ sont des produits connus qui peuvent être préparés par exemple selon le procédé décrit dans Bull. Soc. Chim. France pp. 685-688 (1960) ou dans le brevet français No. 1 260 578, par condensation, selon la réaction de Friedel-Crafts, d'un alkyl-2 benzofuranne et du chlorure de méthoxy-4 benzoyle dans le benzène, à une température comprise entre 0°C et la température ambiante, et en présence de chlorure stannique comme catalyseur suivie d'hydrolyse en présence d'un acide fort tel que l'acide chlorhydrique.

Selon une méthode préférée, on prépare l'alkyl-2 (méthoxy-4 benzoyl)-3 benzofuranne à une température comprise entre -10°C et la température ambiante également selon la réaction de Friedel-Crafts à partir de l'alkyl-2 benzofuranne, d'un halogénure de méthoxy-4 benzoyle, de préférence le chlorure, d'un hydrocarbure aromatique comme solvant par exemple le benzène ou de préférence le toluène et en présence d'un catalyseur, avec hydrolyse ultérieure en présence d'un acide fort par exemple l'acide chlorhydrique. Dans cette variante, cependant on utilise comme catalyseur le chlorure ferrique.

On a pu remarquer, en effet, que ce composé conduit à des résultats supérieurs à ceux procurés par d'autres acides de Lewis tel que le chlorure d'aluminium, le chlorure de zinc, l'éthérate de trifluorure de bore ou le chlorure stannique. Par rapport à ce dernier, les avantages offerts par le chlorure ferrique peuvent se résumer comme suit:

- chargement total du chlorure ferrique dans le réacteur, en une seule opération. Celle-ci se traduit par un gain de main d'oeuvre comparativement au procédé connu qui nécessite, en effet, plusieurs heures pour l'introduction du chlorure stannique
- abaissement du prix de revient d'une part en raison de l'utilisation du chlorure ferrique au lieu du chlorure stannique, d'autre part en raison d'une réduction de 90 % de la quantité d'acide fort engagé pour l'hydrolyse
- gain de rendement dû au fait que l'alkyl-2 (méthoxy-4 benzoyl)-3 benzofuranne peut être utilisé à l'état brut

pour l'obtention des complexes moléculaires de l'invention
- sécurité accrue pour le personnel en raison de l'utilisation du chlorure ferrique moins toxique que le chlorure stannique
- réduction de la teneur en isomères d'alkyl-2 (méthoxy-4 benzoyl)-3 benzofuranne (produits d'acylation en positions 5 et 6 sur l'homocycle) avec accroissement du rendement. Par exemple, lors de la préparation du n-butyl-2 (méthoxy-4 benzoyl)-3 benzofuranne le procédé nécessitant l'emploi de chlorure stannique donne naissance à environ 5 % d'isomères alors que la méthode utilisant le chlorure ferrique ne procure qu'un maximum de 2 % de ces isomères. Un tel avantage est particulièrement intéressant car il évite la purification difficile des alkyl-2 (méthoxy-4 benzoyl)-3 benzofurannes sous forme huileuse.

De manière surprenante, le chlorure ferrique se révèle, dans le cas présent, plus sélectif que le chlorure stannique tout en fournissant des rendements pratiquement quantitatifs.

Les isomères du n-butyl-2 (méthoxy-4 benzoyl)-3 benzofuranne dont il est question ci-dessus, ont été isolés et leur structure déterminée par spectrographie R.M.N. (résonnance magnétique nucléaire) du $^{13}C$.

Il s'agit d'un mélange de n-butyl-2 (méthoxy-4 benzoyl)-6 benzofuranne et de n-butyl-2 (méthoxy-4 benzoyl)-5 benzofuranne dans des proportions approximatives 85 : 15.

Ces impuretés, si elles ne sont pas éliminées, participent aux réactions ultérieures dans lesquelles le n-butyl-2 (méthoxy-4 benzoyl)-3 benzofuranne est engagé et conduiront, par exemple, aux isomères correspondants du n-butyl-2 (hydroxy-4 benzoyl)-3 benzofuranne d'abord puis aux isomères correspondants de l'amiodarone ensuite.

Les complexes moléculaires 2 : 1 de l'invention peuvent être utilisés en vue de la préparation des alkyl-2 (hydroxy-4 benzoyl)-3 benzofuranne.

La solution de complexe moléculaire 2 : 1 de l'invention peut être constituée:

- soit d'une solution extemporanée de complexe 2 : 1 en question dans le solvant choisi,
- soit d'une solution de chlorure d'aluminium et d'alkyl-2 (méthoxy-4 benzoyl)-3 benzofuranne dans un rapport molaire 1 à 2 : 1 également dans le solvant choisi. Dans ce cas, la solution chlorure d'aluminium/alkyl-2 (méthoxy-4 benzoyl)-3 benzofuranne en question peut être préparée elle-même:
- soit par introduction dans le solvant choisi de chlorure d'aluminium anhydre et d'alkyl-2 (méthoxy-4 benzoyl)-3 benzofuranne préalablement isolé de son milieu de préparation
- soit par addition de chlorure d'aluminium anhydre dans une solution d'alkyl-2 (méthoxy-4 benzoyl)-3 benzofuranne brut c'est-à-dire non isolé de son milieu de préparation.

Différents modes de mise en oeuvre des réactifs nécessaires à la préparation de la solution du complexe 2 : 1 selon l'invention et finalement à la réaction de déméthylation ont été testés à savoir:

- chargement de l'ensemble des réactifs à température ambiante et chauffage jusqu'au reflux. Ce mode de réalisation, applicable au laboratoire, s'est révélé dangereux au niveau industriel suite au risque d'emballement de la réaction, soit en raison de l'exothermicité de celle-ci, soit en raison d'un départ trop violent du chlorure de méthyle formé,
- introduction du chlorure d'aluminium dans l'hydrocarbure aromatique, chauffage jusqu'au reflux et coulée progressive, en maintenant le reflux, de la solution d'alkyl-2 (méthoxy-4 benzoyl)-3 benzofuranne.

Cette variante a pour inconvénient de maintenir, dans le milieu réactionnel, un très large excès de chlorure d'aluminium par rapport au dérivé de benzofuranne pendant toute la durée de la coulée, ce qui peut conduire à une désacylation partielle de l'alkyl-2 (méthoxy-4 benzoyl)-3 benzofuranne.

Par contre, on a trouvé que la déméthylation en question peut être réalisée très aisément selon une mise en oeuvre tout à fait particulière.

Ainsi, selon l'invention, on forme à une température inférieure ou égale à la température ambiante, une solution de 1 à 2 moles de chlorure d'aluminium par mole d'alkyl-2 (méthoxy-4 benzoyl)-3 benzofuranne dans l'hydrocarbure aromatique choisi, de manière à former le complexe chlorure d'alumimium/alkyl-2 (méthoxy-4 benzoyl)-3 benzofuranne 1 : 1 de l'invention et on coule la solution obtenue, par exemple une solution à 30 % environ de ce complexe dans le solvant en question maintenu au reflux, ce qui provoque la formation du complexe chlorure d'aluminium/alkyl-2 (hydroxy-4 benzoyl)-3 benzofuranne 2 : 1 de l'invention.

On réalise alors l'hydrolyse de la solution de complexe 2 : 1 ainsi formée comme indiqué précédemment c'est-à-dire en présence d'un acide fort à une température ne dépassant pas 75°C à 80°C.

Selon une méthode connue pour réaliser la déméthylation du n-butyl-2 (méthoxy-4 benzoyl)-3 benzofuranne, on chauffe ce composé à 210 - 220°C en présence de chlorhydrate de pyridine et on hydrolyse en présence d'acide chlorhydrique tel que décrit dans le brevet français No. 1 260 578.

Comparativement à cette méthode connue, la déméthylation de l'alkyl-2 (méthoxy-4 benzoyl)-3 benzofuranne, par l'intermédiaire des complexes moléculaires de l'invention présente plusieurs avantages parmi lesquels on peut citer:

- une température de mise en oeuvre inférieure à celle du procédé connu. De ce fait, la synthèse peut être réalisée dans un appareillage classique permettant d'atteindre par exemple le reflux du toluène sous

pression atmosphérique (110°C) ou sous pression réduite (80°C) tout en évitant l'emploi d'un thermofluide comme moyen de chauffage pour atteindre les conditions de température du procédé antérieur
- l'élimination de l'emploi de pyridine toxique assurant de ce fait un gain de sécurité pour le personnel
- un rendement et une qualité améliorés,
- l'absence de l'impureté présente dans le procédé antérieur à raison de 3 à 10 % selon la teneur en eau du milieu réactionnel. Dans le cas de la déméthylation de l'éthyl-2 (méthoxy-4 benzoyl)-3 benzofuranne, cette impureté correspond à l'(hydroxy-4 phényl)-2 propionyl-3 benzofuranne (P.F.: 171°C) et dans le cas du n-butyl-2 (méthoxy-4 benzoyl)-3 benzofuranne, il s'agit plutôt d'un produit de désacylation à savoir l'(hydroxy-4 phényl)-2 benzofuranne (P.F.: 196°C; spectre I.R.: absence de bande ν C = O, présence de bande OH; spectre R.M.N.: absence de n-$C_4H_9$).

La présence de telles impuretés est particulièrement gênante. En effet, celles-ci nécessitent au minimum une double purification de l'alkyl-2 (hydroxy-4 benzoyl)-3 benzofuranne sans possibilité de recyclage des eaux-mères. Ces impuretés étant généralement plus insolubles que l'alkyl-2 (hydroxy-4 benzoyl)-3 benzofuranne dans les solvants usuels, leur élimination s'avère particulièrement difficile.

Il apparaît en conséquence que les alkyl-2 (hydroxy-4 benzoyl)-3 benzofurannes en question et notamment la benzarone, peuvent être obtenus avec grande facilité et nombreux avantages par l'intermédiaire des complexes moléculaires de l'invention.

Ces avantages sont particulièrement intéressants lorsque les alkyl-2 (méthoxy-4 benzoyl)-3 benzofurannes de départ sont préparés selon la méthode préférée de l'invention c'est-à-dire selon une réaction de Friedel-Crafts avec le chlorure ferrique comme catalyseur. En effet, il est possible de cette manière, de réaliser les différentes étapes de préparation c'est-à-dire la préparation de l'alkyl-2 (méthoxy-4 benzoyl)-3 benzofuranne, la préparation des complexes moléculaires et la déméthylation, dans le même hydrocarbure aromatique, par exemple le toluène, en évitant ainsi l'isolement de l'alkyl-2 (méthoxy-4 benzoyl)-3 benzofuranne intermédiaire.

Ainsi, selon un autre de ses aspects, l'invention se rapporte à la préparation d'alkyl-2 (hydroxy-4 benzoyl)-3 benzofurannes par réaction entre:

- un alkyl-2 benzofuranne et un halogénure de méthoxy-4 benzoyle, de préférence le chlorure, dans un hydrocarbure aromatique, par exemple le toluène, à une température comprise entre -10°C et la température ambiante et en présence de chlorure ferrique comme catalyseur puis hydrolyse en présence d'un acide fort, par exemple l'acide chlorhydrique, pour obtenir une solution d'alkyl-2 (méthoxy-4 benzoyl)-3 benzofuranne correspondant, procédé selon lequel:
- on introduit, à température inférieure ou égale à la température ambiante, 1 à 2 moles de chlorure d'aluminium par mole d'alkyl-2 (méthoxy-4 benzoyl)-3 benzofuranne puis on coule, dans un hydrocarbure aromatique au reflux, par exemple le toluène, la solution ainsi formée,
- on hydrolyse en présence d'un acide fort, par exemple l'acide chlorhydrique, à une température de l'ordre de 75 à 80°C, pour obtenir l'alkyl-2 (hydroxy-4 benzoyl)-3 benzofuranne désiré.

Les alkyl-2 (hydroxy-4 benzoyl)-3 benzofurannes peuvent être utilisés eux-mêmes comme intermédiaires de synthèse pour préparer notamment la benziodarone, la benzbromarone ou l'amiodarone selon des procédés connus.

Par exemple, la benziodarone et la benzbromarone peuvent être obtenues respectivement par iodation ou bromation de l'éthyl-2 (hydroxy-4 benzoyl)-3 benzofuranne au moyen respectivement d'iode ou de brome en phase homogène et en présence d'une solution tampon acétate de métal alcalin/acide acétique et l'amiodarone peut être préparée par iodation du n-butyl-2 (hydroxy-4 benzoyl)-3 benzofuranne, de manière analogue à celle décrite précédemment, pour obtenir le n-butyl-2 (diiodo-3,5 hydroxy-4 benzoyl)-3 benzofuranne suivie d'une éthérification par le chlorhydrate de diéthylamino-1 chloro-2 éthane en présence d'une solution tampon carbonate de métal alcalin/bicarbonare de métal alcalin.

Les Exemples non limitatifs suivants illustrent l'invention:

**Exemple 1**

**Préparation du complexe chlorure d'aluminium/n-butyl-2 (méthoxy-4 benzoyl)-3 benzofuranne 1 : 1**

On dissout 11,5 g (0,0373 mole) de n-butyl-2 (méthoxy-4 benzoyl)-3 benzofuranne dans 100 ml de chlorure de méthylène anhydre. On refroidit la solution à -10°C à l'abri de l'humidité et on y ajoute rapidement et en une fois 5 g (0,0373 mole) de chlorure d'aluminium anhydre. On filtre la solution totale pour éliminer les quelques impuretés en suspension et on évapore à sec sous pression réduite (p = 20 mm Hg; température < 10°C) et sous atmosphère d'azote séché sur ponce sulfurique.

Le miel obtenu ne cristallise pas au congélateur aprés 15 h à -20°C. On le reprend deux fois par 50 ml de tétrachlorure de carbone et on constate, la deuxième fois, une cristallisation après 48 h à -20°C.

On isole et sèche les cristaux sous courant d'azote sec à -20°C et on les conserve au congélateur.

On obtient ainsi 14,8 g de complexe chlorure d'aluminium/n-butyl-2 (méthoxy-4 benzoyl)-3 benzofuranne 1 : 1

sous la forme d'une masse cristalline jaune foncé, peu stable, s'hydrolysant rapidement.

Rendement: 89,7 %

P.F.:translucide à + 25° C

entièrement fondu à + 40° C

non réversible

De la même manière que précédemment, on prépare le complexe chlorure d'aluminium/éthyl-2 (méthoxy-4 benzoyl)-3 benzofuranne 1 : 1.

**Exemple 2**

**Préparation du complexe chlorure d'aluminium/n-butyl-2 (hydroxy-4 benzoyl)-3 benzofuranne 2 : 1**

On dissout 30,8 g (0,1 mole) de n-butyl-2 (méthoxy-4 benzoyl)-3 benzofuranne dans 300 ml de toluène sec et on refroidit à -10°C à l'abri de l'humidité. On ajoute alors rapidement et en une fois 26,7 g (0,2 mole) de chlorure d'aluminium anhydre. On obtient ainsi une solution contenant le complexe chlorure d'aluminium/n-butyl-2 (méthoxy-4 benzoyl)-3 benzofuranne 1 : 1, que l'on coule dans du toluène anhydre porté à la température de reflux. On maintient encore la température de reflux pendant une heure tout en distillant du toluène.

En fin de réaction, on refroidit progressivement le milieu réactionnel. La cristallisation s'amorce vers +40° C. On essore alors après 2 h de maintien à +5° C et on sèche le cake d'essorage en étuve à vide à +55° C sous courant d'azote sec.

On obtient ainsi 49 g de complexe chlorure d'aluminium/n-butyl-2 (hydroxy-4 benzoyl)-3 benzofuranne 2 : 1 sous forme d'une poudre jaûnatre finement divisée.

P.F.: > 300° C avec décomposition

Spectre I.R.: absence de bande C = O libre, bande C = O associée

Spectre R.M.N.: spectre très large, absence de signal $-OCH_3$

Dosage de l'aluminium (complexométrie): 9,36 - 9,5 - 9,6 % (théorie: 9,63 %).

De la même manière que précédemment, on prépare le complexe chlorure d'aluminium/éthyl-2 (hydroxy-4 benzoyl)-3 benzofuranne 2 : 1.

**Exemple 3**

**Préparation de solutions contenant les complexes chlorure d'aluminium/n-butyl-2 (méthoxy-4 et hydroxy-4 benzoyl)-3 benzofurannes.**

a) n-Butyl-2 (méthoxy-4 benzoyl)-3 benzofuranne

Dans un réacteur, on introduit 520 g (600 ml) de toluène et 174 g (1 mole) de n-butyl-2 benzofuranne. Sous agitation, on porte le mélange au reflux et on sèche le milieu et l'appareillage par étêtage au moyen d'un système Dean-Stark. On élimine ainsi par distillation environ 35 g (environ 40 ml) de toluène. On refroidit à température ambiante et on introduit rapidement en une fois, 174 g (1,02 mole) de chlorure de méthoxy-4 benzoyle. On glace à -10° ± 2° C et on introduit également rapidement et en une fois 130 g (0,8 mole) de chlorure ferrique anhydre.

On maintient le milieu réactionnel à -10° ± 2° C pendant 15 min. puis on laisse revenir spontanément à température ambiante. On maintient pendant 6 heures sous agitation et on glace à nouveau vers 0° C. On introduit alors, progressivement 200 g d'eau épurée sans dépasser 20° C.

On maintient 30 min. à température ambiante, on filtre l'insoluble (acide méthoxy-4 benzoïque) puis on rince sur le filtre avec 90 g de toluène. On réunit les filtrats dans l'ampoule, décante la phase aqueuse inférieure et lave la couche organique avec une solution de 30 g d'acide chlorhydrique à 36 % dans 200 g d'eau épurée puis avec de l'eau jusqu'à neutralité. On sèche la phase organique et on élimine le solvant sous vide jusqu'à une température de 80° C dans la masse sous pression résiduelle d'environ 50 mm Hg.

On obtient ainsi environ 308 g de n-butyl-2 (méthoxy-4 benzoyl)-3 benzofuranne sous forme brute.

Rendement: environ 100 %

Le n-butyl-2 (méthoxy-4 benzoyl)-3 benzofuranne obtenu selon cette méthode conduit en moyenne aux teneurs suivantes en impuretés connues par chromatographie sur couche mince.

| | |
|---|---|
| n-Butyl-2 benzofuranne | ≤ 1,5 % |
| n-Butyl-2 (méthoxy-4 benzoyl)-6 benzofuranne/ n-butyl-2 (méthoxy-4 benzoyl)-5 benzofuranne | ≤ 2 % |

b) Complexes chlorure d'aluminium/n-butyl-2 (méthoxy-4 et hydroxy-4 benzoyl)-3 benzofurannes

Dans un réacteur muni de dispositifs d'introduction de réactifs, d'agitation et de réfrigération, on introduit

308,3 g (1 mole) de n-butyl-2 (méthoxy-4 benzoyl)-3 benzofuranne dans 625 g de toluène. Après refroidissement du mélange à 0° C, on ajoute, sous agitation rapide, 253,3 g (1,9 mole) de chlorure d'aluminium.

On obtient ainsi une solution contenant le complexe chlorure d'aluminium/n-butyl-2 (méthoxy-4 benzoyl)-3 benzofuranne 1 : 1.

Dans un second réacteur identique renfermant 493 g de toluène anhydre porté à la température de reflux, on introduit la solution toluénique contenant le complexe 1 : 1 ainsi préparée. On maintient le reflux du mélange réactionnel encore pendant une heure après la fin de l'addition tout en distillant du toluène (462 g), ce qui provoque la formation du complexe chlorure d'aluminium/n-butyl-2 (hydroxy-4 benzoyl)-3 benzofuranne 2 : 1.

On obtient ainsi une solution toluénique de complexe moléculaire chlorure d'aluminium/n-butyl-2 (hydroxy-4 benzoyl)-3 benzofuranne que l'on utilise telle quelle.

**Exemple 4**

**Préparation de solutions contenant les complexes chlorure d'aluminium/n-butyl-2 (méthoxy-4 et hydroxy-4 benzoyl)-3 benzofurannes.**

a) n-Butyl-2 (méthoxy-4 benzoyl)-3 benzofuranne

Dans un réacteur muni de dispositifs d'agitation, d'introduction de réactifs et d'un réfrigérant, on introduit 174 g (1 mole) de n-butyl-2 benzofuranne et 520 g de toluène. On entraine les traces d'eau par distillation azéotropique. On refroidit le mélange à 20° C et on ajoute rapidement 174 g (1,02 mole) de chlorure de méthoxy-4 benzoyle. Après refroidissement à -10° C, on traite le milieu par 130 g de chlorure ferrique ajoutés en une fois. Après 15 min. à cette température, on laisse le mélange revenir à la température ambiante et on l'y maintient pendant 6 h sous agitation. On hydrolyse par addition de 200 ml d'eau en ne laissant pas la température dépasser 20° C. Après 15 min. à cette température, on filtre un insoluble (acide méthoxy-4 benzoïque), on lave le filtre avec du toluène et on sépare la phase aqueuse. On lave la phase organique avec une solution de 30 g d'acide chlorhydrique à 36 % dans 200 ml d'eau ensuite avec de l'eau jusqu'à neutralité puis on sèche.

On obtient ainsi une solution toluénique contenant environ 308 g (environ 100 %) de n-butyl-2 (méthoxy-4 benzoyl)-3 benzofuranne que l'on utilise telle quelle.

b) Complexes chlorure d'aluminium/n-butyl-2 (méthoxy-4 et hydroxy-4 benzoyl)-3 benzofurannes.

Dans un réacteur, on introduit une solution toluénique de 308,3 g de n-butyl-2 (méthoxy-4 benzoyl)-3 benzofuranne brut (exprimé en extrait sec: 1 mole) telle qu'obtenue au paragraphe a) ci-dessus, c'est-à-dire une solution à environ 30 %.

Sous agitation, on ajoute rapidement en refroidissant à 0° C, 253,3 g (1,9 mole) de chlorure d'aluminium et on laisse revenir à température ambiante.

On obtient ainsi une solution toluénique contenant le complexe chlorure d'aluminium/n-butyl-2 (méthoxy-4 benzoyl)-3 benzofuranne 1 : 1.

Dans un second réacteur, on introduit 493 g de toluène, puis, sous agitation, on porte à reflux et on sèche l'appareillage et le solvant par azéotropie. En maintenant par chauffage le reflux du milieu, on ajoute en environ 2,5 ± 0,5 h la solution contenant le complexe 1 : 1 obtenue précédemment. On rince avec 138 g de toluène anhydre et on maintient 1 h à reflux franc tout en distillant du toluène (462 g) ce qui provoque la formation du complexe chlorure d'aluminium/n-butyl-2 (hydroxy-4 benzoyl)-3 benzofuranne 2 : 1.

On obtient ainsi une solution toluénique de complexe moléculaire chlorure d'aluminium/n-butyl-2 (hydroxy-4 benzoyl)-3 benzofuranne que l'on utilise telle quelle.

**Exemple 5**

**Préparation de solutions contenant les complexes chlorure d'aluminium/éthyl-2 (méthoxy-4 et hydroxy-4 benzoyl)-3 benzofurannes.**

a) Ethyl-2 (méthoxy-4 benzoyl)-3 benzofuranne

Dans un réacteur, on introduit 146,2 g (1 mole) d'éthyl-2 benzofuranne (titre 99,8 %) et 520 g (600 ml) de toluène. Sous agitation, on porte le milieu au reflux et on sèche par étêtage au moyen d'un système Dean-Stark. On distille ainsi environ 35 g (environ 40 ml) de toluène. On refroidit à température ambiante et on ajoute rapidement en une fois 174 g (1,02 mole) de chlorure de méthoxy-4 benzoyle (titre 98 %). On glace à -10 ± 2° C et on introduit rapidement et en une fois 130 g (0,8 mole) de chlorure ferrique anhydre. On maintient le milieu réactionnel à cette température pendant 15 min. puis on laisse revenir spontanément à la température ambiante pendant 6 heures. On glace à 0° C et hydrolyse sans dépasser 10° C par ajout progressif de 200 g d'eau désionisée. On filtre l'insoluble (acide méthoxy-4 benzoïque) et on décante la phase aqueuse. On lave la phase organique avec une solution préalablement préparée, de 200 g d'eau désionisée et de 30 g d'acide

chlorhydrique à 36 % puis à l'eau désionisée jusqu'à pH 5 à 6 de l'effluent. On distille environ 500 g de toluène sous pression réduite (température de la masse: 80°C, pression résiduelle: 80 à 100 mm Hg) puis on casse le vide à l'azote et on introduit 280 g de n-heptane. On cristallise à température ambiante, essore après 2h de glaçage à -5 à -10°C puis on sèche à poids constant en étuve ventilée à 50°C.

On obtient ainsi environ 260 g d'éthyl-2 (méthoxy-4 benzoyl)-3 benzofuranne brut.
Rendement: environ 93 %
P.F.: 81°C

b) Complexes chlorure d'aluminium/éthyl-2 (méthoxy-4 et hydroxy-4 benzoyl)-3 benzofurannes

Dans un premier réacteur, on introduit 280,3 g (1 mole) d'éthyl-2 (méthoxy-4 benzoyl)-3 benzofuranne cristallisé et 588 g de toluène. Sous agitation, on dissout par chauffage, sèche les réactifs par azéotropie et refroidit la solution obtenue à 10°C.

Dans un second réacteur parfaitement sec, on introduit 253 g de toluène anhydre puis, sous refroidissement, 241 g (1,8 mole) de chlorure d'aluminium anhydre. Om amène la température de masse à 0 ± 5°C et on ajoute la solution toluénique d'éthyl-2 (méthoxy-4 benzoyl)-3 benzofuranne préparée dans le premier réacteur. L'addition se fait en 90 min. environ sous refroidissement extérieur pour ne pas dépasser 15°C. On maintient alors à température inférieure ou égale à 15°C la solution contenant le complexe chlorure d'aluminium/éthyl-2 (méthoxy-4 benzoyl)-3 benzofuranne 1 : 1 ainsi formée.

Dans le premier réacteur, on introduit 356 g de toluène anhydre puis on place sous agitation et sous pression résiduelle de 300 - 350 mm Hg avant de porter le toluène au reflux (température de masse: 84 ± 1°C). On stabilise le vide et la température, on met l'installation en distillation puis on commence d'introduire régulièrement la solution du second réacteur contenant le complexe (temps total d'introduction du complexe: 4 à 5 h; distillation toluénique: environ 1400 g). On rince le second réacteur avec 48 g de toluène anhydre et on porte la masse à 100°C en réduisant progressivement la dépression dans le réacteur (distillation toluénique: environ 105 g) ce qui provoque la formation de complexe chlorure d'aluminium/éthyl-2 (hydroxy-4 benzoyl)-3 benzofuranne 2 : 1.

On obtient ainsi une solution toluénique de complexe moléculaire chlorure d'aluminium/éthyl-2 (hydroxy-4 benzoyl)-3 benzofuranne.

**Exemple 6**

**Préparation de solutions contenant les complexes chlorure d'aluminium/éthyl-2 (méthoxy-4 et hydroxy-4 benzoyl)-3 benzofurannes.**

a) Ethyl-2 (méthoxy-4 benzoyl)-3 benzofuranne.

Dans un réacteur, on introduit 109,6 g (0,75 mole) d'éthyl-2 benzofuranne et 388 g de toluène. On sèche le milieu par azéotropie (distillat toluénique: environ 27 g). On refroidit à 20°C et on ajoute rapidement 130 g de chlorure de méthoxy-4 benzoyle. On refroidit à -10 ± 2°C et on ajoute rapidement 97 g de chlorure ferrique anhydre. On maintient durant 30 min. à basse température puis on laisse revenir à la température de 20 ± 3°C que l'on maintient durant 6 h. On hydrolyse par ajout de 150 g d'eau désionisée, on porte la masse à 40 ± 2°C et on décante la phase aqueuse. On lave la phase organique à 40 ± 2°C pendant 30 min. avec une solution, préalablement préparée, de 150 g d'eau désionisée et de 225 g d'acide chlorhydrique à 36 %. On décante et on lave toujours à 40°C, avec 3 à 5 fractions de 200 g d'eau désionisée jusqu'à pH ⩾ 5 de l'effluent. On traite la solution toluénique par 45 g de charbon actif. On porte au reflux et on élimine l'eau par azéotropie. On filtre à chaud pour éliminer les impuretés, rince toujours à chaud avec 68 g de toluène et réunit les filtrats.

On obtient ainsi une solution toluénique contenant environ 190 g (environ: 90 %) d'éthyl-2 (méthoxy-4 benzoyl)-3 benzofuranne que l'on utilise telle quelle.

b) Complexes chlorure d'aluminium/éthyl-2 (méthoxy-4 et hydroxy-4 benzoyl)-3 benzofurannes.

Dans un premier réacteur parfaitement sec, on introduit une solution toluénique de 280,3 g d'éthyl-2 (méthoxy-4 benzoyl)-3 benzofuranne brut (exprimé en extrait sec = 1 mole) telle qu'obtenue au paragraphe a) précédent, c'est-à-dire une solution à environ 30 %. On refroidit à température inférieure ou égale à 5°C et, sous agitation, on ajoute rapidement et en une fois 266 g (2 moles) de chlorure d'aluminium anhydre pour former une solution contenant le complexe chlorure d'aluminium/éthyl-2 (méthoxy-4 benzoyl)-3 benzofuranne 1 : 1. On maintient cette solution contenant le complexe sous agitation durant 30 min. à température inférieure à 30°C et à l'abri de l'humidité.

Dans un second réacteur, on porte au reflux 348 g (400 ml) de toluène anhydre puis, sous reflux, on introduit en 1,5 à 2 h la solution toluénique contenant le complexe obtenue précédemment. Dès la fin d'introduction, on met l'appareil en distillation (distillat toluénique: environ 400 ml) et on place à nouveau sous reflux total durant 1 h, ce qui provoque la formation de complexe chlorure d'aluminium/éthyl-2 (hydroxy-4 benzoyl)-3 benzofuranne 2 : 1.

On obtient ainsi une solution toluénique de complexe moléculaire chlorure d'aluminium/éthyl-2 (hydroxy-4 benzoyl)-3 benzofuranne que l'on utilise telle quelle.

**Exemple 7**

**Préparation de solutions contenant les complexes chlorure d'aluminium/éthyl-2 (méthoxy-4 et hydroxy-4 benzoyl)-3 benzofurannes.**

Dans un réacteur parfaitement sec, on introduit 244 g (280 ml) de toluène anhydre puis rapidement en une fois 266 g (2 moles) de chlorure d'aluminium anhydre. Sous agitation et en refroidissant pour ne pas dépasser 20°C, on ajoute progressivement une solution toluénique de 280,3 g d'éthyl-2 (méthoxy-4 benzoyl)-3 benzofuranne brut (exprimé en extrait sec = 1 mole) telle qu'obtenue à l'Exemple 6a) c'est-à-dire une solution à environ 30 %. On porte progressivement au reflux en 1 à 1,5 h puis on maintient 1 h à cette température.

On obtient ainsi une solution toluénique de complexe moléculaire chlorure d'aluminium/éthyl-2 (hydroxy-4 benzoyl)-3 benzofuranne 2 : 1.

De la même manière que celle décrite précédemment mais à partir d'une solution toluénique de 308,3 g de n-butyl-2 (méthoxy-4 benzoyl)-3 benzofuranne (exprimé en extrait sec: 1 mole), on obtient une solution toluénique de complexe moléculaire chlorure d'aluminium/n-butyl-2 (hydroxy-4 benzoyl)-3 benzofuranne 2 : 1.

Les Exemples suivants illustrent la préparation de dérivés de benzoyl-3 benzofuranne et notamment la benzarone, la benziodarone, la benzbromarone et l'amiodarone à partir des complexes moléculaires de formule I de l'invention.

**Exemple I**

**Préparation du n-butyl-2 (hydroxy-4 benzoyl)-3 benzofuranne.**

Dans une solution de 46,2 g d'acide chlorhydrique à 36 % dans 600 g d'eau, on verse, sous agitation, la solution toluénique de complexe moléculaire obtenue à l'Exemple 3b), refroidie vers 75°C. Après 15 min., on sépare le phase aqueuse et on lave la phase organique jusqu'à neutralité avec de l'eau. On sèche la phase toluénique par distillation azéotropique de l'eau (distillation de 92 g de toluène) et on la décolore par traitement à 80°C avec de la terre décolorante. Après filtration et rinçage du filtre avec du toluène, on refroidit le filtrat vers -5° à -10°C et on isole le produit désiré par essorage.

On obtient ainsi 253 g de n-butyl-2 (hydroxy-4 benzoyl)-3 benzofuranne brut.

Rendement: 86 %

P.F.: 119°C

Une chromatographie sur couche mince montre que ce composé est exempt d'(hydroxy-4 phényl)-2 benzofuranne.

**Exemple II**

**Préparation de l'éthyl-2 (hydroxy-4 benzoyl)-3 benzofuranne**

A une solution de 108 g d'acide chlorhydrique à 36 % dans 530 g d'eau désionisée, on ajoute, en 1 à 2 h et sans dépasser 80°C, la solution toluénique de complexe moléculaire, obtenue à l'Exemple 5b), préalablement refroidie à 75°C. On décante la phase aqueuse inférieure à 75 - 80°C et on lave la couche organique à cette température avec 3 fractions chacune de 280 g d'eau désionisée. Après la dernière décantation, on sèche le milieu par azéotropie à pression normale par l'intermédiaire d'un système Dean-Stark jusqu'à obtention d'une température de tête de 108 - 110°C. On refroidit à 80°C et on traite durant 15 min. avec 11 g de terre décolorante. On filtre et rince à chaud avec 60 g de toluène. On réunit les filtrats, cristallise par refroidissement progressif et glace durant 2 h à -5°C avant d'essorer. On rince avec 2 fractions chacune de 120 g de toluène anhydre et glacé et on sèche à poids constant en étuve à vide à 50°C.

On obtient ainsi environ 233 g d'éthyl-2 (hydroxy-4 benzoyl)-3 benzofuranne brut ou benzarone.

Rendement: 83 ± 3 % [par rapport à l'éthyl-2 (méthoxy-4 benzoyl]-3 benzofuranne).

**Exemple III**

**Préparation de l'éthyl-2 (hydroxy-4 benzoyl)-3 benzofuranne.**

Sous agitation, on introduit dans 600 g d'eau désionisée, la solution toluénique de complexe moléculaire, obtenue à l'Exemple 6b), préalablement refroidie à 70 ± 5° C. On porte à nouveau rapidement au reflux puis on refroidit à 70 ± 5° C pour décanter la couche aqueuse inférieure. On lave la couche organique toujours à 70 ± 5° C avec une solution de 42 g d'acide chlorhydrique à 36 % dans 266 g d'eau désionisée puis jusqu'à neutralité avec 4 fractions chacune de 266 g d'eau désionisée. On sèche la couche organique par azéotropie à pression normale (distillat toluène/eau: environ 244 g ou 280 ml) et on traite le milieu obtenu avec 84 g de terre décolorante pendant 15 min. à 80° C. On filtre, on rince à chaud avec 56 g (65 ml) de toluène et on réunit les filtrats. On cristallise par refroidissement et on essore après 2 h à -5 à -10° C. On rince avec 168 g (195 ml) de toluène glacé et on sèche à poids constant en étuve ventilée à 60° C.

On obtient ainsi environ 234 g d'éthyl-2 (hydroxy-4 benzoyl)-3 benzofuranne brut ou benzarone.

Rendement: environ 88 % [par rapport à l'extrait sec exprimé en éthyl-2 (méthoxy-4 benzoyl)-3 benzofuranne].

**Exemple IV**

**Préparation de l'éthyl-2 (hydroxy-4 benzoyl)-3 benzofuranne**

Sous agitation, on introduit dans 600 g d'eau désionisée la solution toluénique de complexe moléculaire, obtenue à l'Exemple 7, préalablement refroidie à 80 ± 5° C. On porte à nouveau rapidement au reflux, puis on refroidit à 70 ± 5° C pour decanter la couche aqueuse inférieure. On lave la couche organique toujours à 70 ± 5° C avec une solution de 42 g d'acide chlorhydrique à 36 % dans 266 g d'eau désionisée puis jusqu'à neutralité avec 4 fractions chacune de 266 g d'eau désionisée. On sèche la couche organique par azéotropie à pression normale (distillat toluène/eau: environ 244 g ou 280 ml) et on traite le milieu obtenu avec 84 g de terre décolorante pendant 15 min. à 80° C. On filtre, on rince à chaud avec 56 g (65 ml) de toluène et on réunit les filtrats. On cristallise par refroidissement et on essore après 2h à -5 à -10° C. On rince avec 168 g (195 ml) de toluène glacé et on sèche à poids constant en étuve ventilée à 60° C.

On obtient ainsi environ 266 g d'éthyl-2 (hydroxy-4 benzoyl)-3 benzofuranne brut ou benzarone.

Rendement: 88 ± 1 % [par rapport à l'extrait sec exprimé en ethyl-2 (méthoxy-4 benzoyl)-3 benzofuranne].

P.F.: 126° C.

De la même manière que celle décrite précédemment mais au départ de la solution toluénique de complexe moléculaire obtenue à l'Exemple 8, on obtient environ 216 g d'éthyl-2 (hydroxy-4 benzoyl)-3 benzofuranne brut, ce qui représente un rendement de 81 ± 1 % par rapport à l'extrait sec exprimé en éthyl-2 (méthoxy-4 benzoyl)-3 benzofuranne.

P.F.: 125° C.

**Exemple V**

**Préparation du chlorhydrate de n-butyl-2 (diiodo-3,5 β-diéthylaminoéthoxy-4 benzoyl)-3 benzofuranne.**

a) n-Butyl-2 (hydroxy-4 benzoyl)-3 benzofuranne

Sous agitation et à température inférieure ou égale à 75° C, on hydrolyse la solution toluénique de complexe moléculaire obtenue à l'Exemple 4b) par addition d'une solution de 46,2 g d'acide chlorhydrique à 36 % dans 600 g d'eau distillée. On rince l'appareillage avec 123 g de toluène que l'on ajoute ensuite au milieu d'hydrolyse. Après 15 min. à la température de 70° C dans la masse, on décante et on lave la couche organique, à 70° C avec des fractions de 308 g d'eau épurée jusqu'à neutralité. On sèche la couche organique par azéotropie tout en distillant environ 92 g de toluène.

On traite le milieu à 80° C avec 12,3 g de terre décolorante, filtre et rince avec 92 g de toluène chaud. On réunit les filtrats, refroidit à 35° C et attend que la cristallisation soit bien amorcée avant de glacer à -5 à -10° C. On maintient 2 h à cette température, essore, rince avec 200 g de toluène glacé et sèche à poids constant en étuve ventilée à 60° C.

On obtient ainsi environ 253 g de n-butyl-2 (hydroxy-4 benzoyl)-3 benzofuranne brut.

Rendement: environ 86 % (par rapport au n-butyl-2 benzofuranne).

b) n-Butyl-2 (diiodo-3,5 hydroxy-4 benzoyl)-3 benzofuranne

Dans un réacteur de 41, on introduit 540 g de méthanol. Sous agitation, on ajoute alors successivement 300 g (2,4 moles) d'acétate de sodium trihydraté, 286 g d'iode et 424 g d'iode récupéré humide (2,3 moles au total). On amène le milieu réactionnel à 30 à 35° C et on introduit en une fois 294 g (1 mole) de n-butyl-2 (hydroxy-4 benzoyl)-3 benzofuranne. On rince avec 60 g de méthanol et on porte le mélange au reflux en 30 à 45 min.

(température dans la masse: 70 à 74°C). On arrête le chauffage et on introduit, en 10 min. environ, une solution préalablement préparée de 90 g (2,2 moles) d'hydroxyde de sodium en écailles dans 400 g d'eau épurée. L'exothermicité de la réaction maintient le reflux (76/77°C dans la masse) pendant le temps d'introduction. On maintient 2 h à reflux puis on modifie l'appareil en distillation à pression atmosphérique. On introduit, en 20 min. environ, une solution aqueuse de 320 g de bisulfite de sodium (35°B) et on poursuit la distillation jusqu'à atteindre 97 à 100°C dans la masse (température de tête: 87°C). On distille ainsi environ 800 ml (environ 680 g) de solvant. On refroidit au moyen d'un bain d'eau à 75 à 80°C et on introduit successivement et dans l'ordre 200 g d'eau épurée, 215 g d'acide chlorhydrique à 36 % et 1600 g de toluène. On porte au reflux pendant 10 min. (température de la masse: 84°C - dégagement d'anhydride sulfurique) et on décante la phase aqueuse inférieure. On lave la couche toluénique à 75 à 80°C successivement avec 400 g d'eau épurée, 100 g d'une solution aqueuse de bisulfite de sodium et 2 fractions chacune de 400 g d'eau épurée. On décante au maximum après le dernier lavage et on traite avec 22 g de charbon actif durant 30 min. au reflux. On filtre à chaud, on rince avec 380 g de toluène chaud et on réunit les filtrats.

On obtient ainsi une solution toluénique de n-butyl-2 (diiodo-3,5 hydroxy-4 benzoyl)-3 benzofuranne que l'on utilise telle quelle.

De la même manière que celle décrite précédemment mais à partir de 266,3 g (1 mole) d'éthyl-2 (hydroxy-4 benzoyl)-3 benzofuranne, on obtient l'éthyl-2 (diiodo-3,5 hydroxy-4 benzoyl)-3 benzofuranne ou benziodarone après élimination du toluène par distillation.

De même, à partir de 266,3 g (1 mole) d'éthyl-2 (hydroxy-4 benzoyl)-3 benzofuranne et de brome, on obtient l'éthyl-2 (dibromo-3,5 hydroxy-4 benzoyl)-3 benzofuranne ou benzbromarone après élimination du toluène par distillation.

c) n-Butyl-2 (diiodo-3,5 β-diéthylaminoéthoxy-4 benzoyl)-3 benzofuranne

Dans un réacteur, on introduit la solution toluénique de n-butyl-2 (diiodo-3,5 hydroxy-4 benzoyl)-3 benzofuranne obtenue au paragraphe b) ainsi que 800 g d'eau épurée et 177,3 g (1,03 mole) de chlorhydrate de diéthylamino-1 chloro-2 éthane. Sous agitation, on amène le milieu réactionnel à 40 ± 2°C et on l'y maintient durant 15 min. En contrôlant le départ d'anhydride carbonique, on introduit alors lentement en pluie 416 g (3 moles) de carbonate de potassium anhydre. On élève progressivement la température pour atteindre le reflux en 1 h. On maintient durant 3 h à cette température, on décante la couche aqueuse saline à 75 ± 5°C et on lave la couche toluénique, à cette température avec 4 fractions chacune de 800 g d'eau épurée. A la température de 60°C on traite la solution toluénique avec 20,5 g de charbon actif, on filtre, on rince avec environ 220 g de toluène et on réunit les filtrats.

On obtient ainsi une solution toluénique de n-butyl-2 (diiodo-3,5 β-diéthylaminoéthoxy-4 benzoyl)-3 benzofuranne sous forme de base ou amiodarone.

d) Chlorhydrate de n-butyl-2 (diiodo-3,5 β-diéthylaminoéthoxy-4 benzoyl)-3 benzofuranne

On amène à 60°C la solution de n-butyl-2 (diiodo-3,5 β-diéthylaminoéthoxy-4 benzoyl)-3 benzofuranne obtenue au paragraphe c) et on introduit en 45 min. 38,5 g de gaz chlorhydrique au moyen d'un tube plongeant. On laisse la température de la masse s'élever suite à l'exothermicité de la réaction sans toutefois dépasser 75°C.

On vérifie le pH franchement acide en fin d'introduction et après 30 min. de contact à 70 ± 5°C. On met progressivement sous vide de la trompe à eau et on distille environ 400 ml de toluène/eau/acide chlorhydrique (fin de distillation: pression résiduelle ⩽ 150 mm; température de la masse ⩽ 75°C). On cristallise sous agitation lente avec bain d'eau durant environ 8 heures et on essore à 10 à 15°C. On rince avec 4 fractions chacune de 180 ml de toluène filtré et on sèche jusqu'à poids constant en étuve ventilée à 60°C.

On obtient ainsi environ 647,5g de chlorhydrate de n-butyl-2 (diiodo-3,5 β-diéthylaminoéthoxy-4 benzoyl)-3 benzofuranne ou chlorhydrate d'amiodarone.

Rendement: environ 95 % [par rapport au n-butyl-2 (hydroxy-4 benzoyl)-3 benzofuranne].

**Revendications** pour les Etats Contractants: BE, CH, DE, FR, GB, IT, LI, LU, NL, SE

1. Complexes moléculaires correspondant à la formule générale:

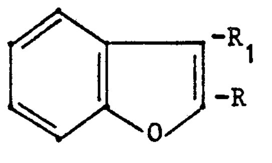

I

dans laquelle R représente un radical alkyle et $R_1$ représente l'un des radicaux:

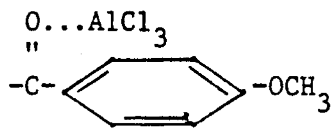

ou

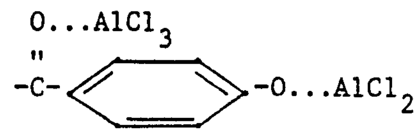

2. Complexes moléculaires selon la Revendication 1, caractérisés en ce que R représente un groupe éthyle ou un groupe n-butyle.

3. Procédé de préparation de complexes moléculaires selon une des Revendications 1 ou 2 de formule I dans laquelle $R_1$ représente le radical:

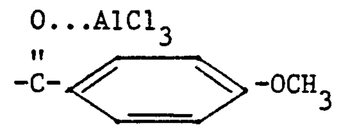

caractérisé en ce que l'on fait réagir le chlorure d'aluminium et un alkyl-2 (méthoxy-4 benzoyl)-3 benzofuranne de formule générale:

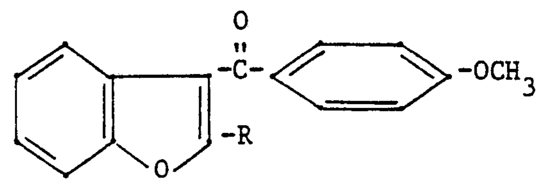

dans laquelle R représente un radical alkyle, dans le rapport molaire 1 à 2 : 1, en milieu hydrocarbure aromatique ou en milieu chloroalkane et à une température inférieure ou égale à la température ambiante.

4. Procédé de préparation de complexes moléculaires selon une des Revendications 1 ou 2 de formule I, dans laquelle $R_1$ représente le radical

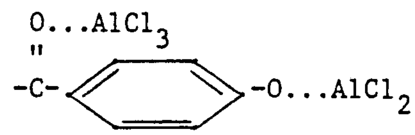

caractérisé en ce que l'on porte au reflux une solution contenant le complexe moléculaire selon une des Revendications 1 ou 2 de formule dans laquelle $R_1$ représente le radical

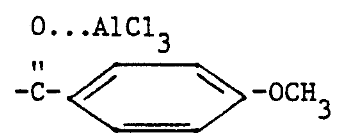

solution formée à température inférieure ou égale à la température ambiante au départ d'un hydrocarbure aromatique et d'une à deux moles de chlorure d'aluminium par mole d'alkyl-2 (méthoxy-4 benzoyl)-3 benzofuranne de formule générale:

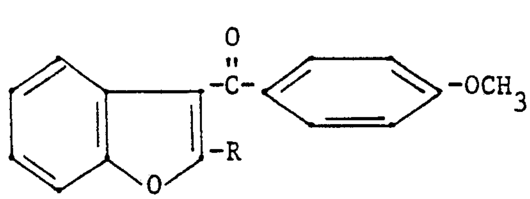

dans laquelle R représente un radical alkyle.

5. Procédé selon la Revendication 4, caractérisé en ce que, dans un hydrocarbure aromatique au reflux, on coule une solution formée à température inférieure ou égale à la température ambiante par un hydrocarbure aromatique et une à deux moles de chlorure d'aluminium par mole d'alkyl-2 (méthoxy-4 benzoyl)-3 benzofuranne.

6. Solutions contenant les complexes moléculaires selon une des Revendications 1 ou 2, caractérisées en ce qu'elles sont formées au départ d'une à deux moles de chlorure d'aluminium par mole d'un alkyl-2 (méthoxy-4 benzoyl)-3 benzofuranne de formule générale:

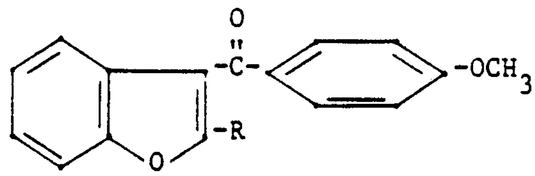

dans laquelle R représente un radical alkyle, et d'un hydrocarbure aromatique.

7. Procédé de préparation de solutions contenant les complexes moléculaires selon une des Revendications 1 ou 2, de formule I, dans laquelle $R_1$ représente le radical

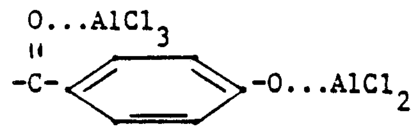

caractérisé en ce que dans un hydrocarbure aromatique au reflux, on coule une solution formée, à température inférieure ou égale à la température ambiante, par un hydrocarbure aromatique et une à deux moles de chlorure d'aluminium par mole d'un alkyl-2 (méthoxy-4 benzoyl)-3 benzofuranne.

8. Procédé de préparation d'alkyl-2 (hydroxy-4 benzoyl)-3 benzofuranne de formule générale:

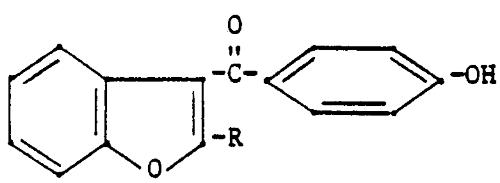

dans laquelle R représente un radical alkyle, selon lequel on fait réagir un alkyl-2 benzofuranne de formule générale:

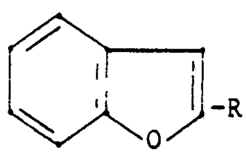

dans laquelle R a la même signification que précédemment, et un halogénure de méthoxy-4 benzoyle, à une température comprise entre -10°C et la température ambiante et en présence de chlorure ferrique comme catalyseur puis on hydrolyse en présence d'un acide fort pour obtenir une solution d'alkyl-2 (méthoxy-4 benzoyl)-3 benzofuranne correspondant, caractérisé en ce qu':

on introduit, à température inférieure ou égale à la température ambiante 1 à 2 moles de chlorure d'aluminium par mole d'alkyl-2 (méthoxy-4 benzoyl)-3 benzofuranne puis on coule, dans un hydrocarbure aromatique au reflux, la solution ainsi formée,

- on hydrolyse en présence d'un acide fort à une température de l'ordre de 75 à 80°C, pour obtenir l'alkyl-2 (hydroxy-4 benzoyl)-3 benzofuranne désiré.

9. Procédé selon l'une quelconque des Revendications 3, 4, 5, 7 ou 8, caractérisé en ce que l'hydrocarbure aromatique est le toluène.

**Revendications** pour l'Etat Contractant: AT

1. Procédé de préparation de complexes moléculaires correspondant à la formule générale:

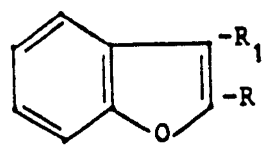 I

dans laquelle R représente un radical alkyle et $R_1$ représente le radical

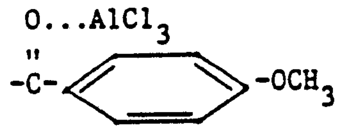

caractérisé en ce que l'on fait réagir le chlorure d'aluminium et un alkyl-2 (méthoxy-4 benzoyl)-3 benzofuranne de formule générale:

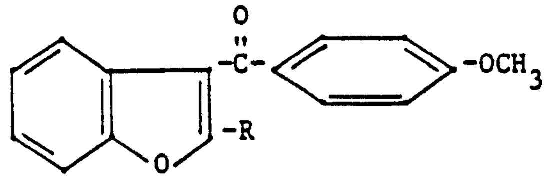

dans laquelle R représente un radical alkyle, dans le rapport molaire 1 à 2 : 1, en milieu hydrocarbure aromatique ou en milieu chloroalkane et à une température inférieure ou égale à la température ambiante.

2. Procédé de préparation de complexes moléculaires correspondant à la formule générale:

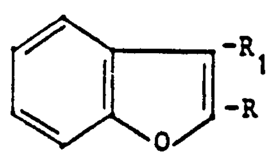

dans laquelle R représente un radical alkyle et $R_1$ représente le radical

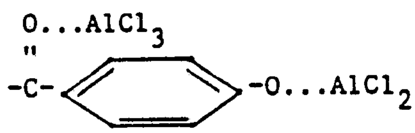

caractérisé en ce que l'on porte au reflux une solution contenant le complexe moléculaire de formule I dans laquelle $R_1$ représente le radical

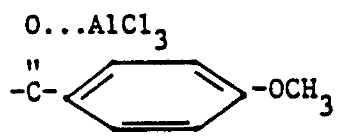

solution formée à temperature inferieure ou égale à la température ambiante au départ d'un hydrocarbure aromatique et d'une à deux moles de chlorure d'aluminium par mole d'alkyl-2 (méthoxy-t benzoyl)-3 benzofuranne de formule générale:

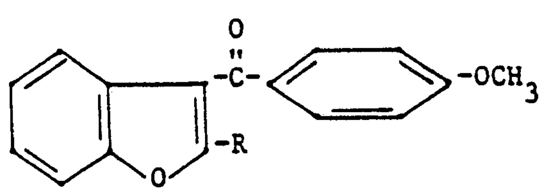

dans laquelle R représente un radical alkyle.

3. Procédé selon la revendication 2, caractérise en ce que, dans un hydrocarbure aromatique au reflux, on coule une solution formée à température inférieure ou égale à la température ambiante par un hydrocarbure

aromatique et une à deux moles de chlorure d'aluminium par mole d'alkyl-2 (méthoxy-4 benzoyl)-3 benzofuranne.

4. Solutions contenant les complexes moléculaires correspondant à la formule générale:

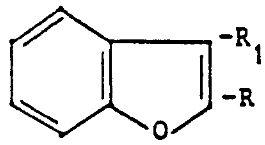

I

dans laquelle R représente un radical alkyle et $R_1$ représente l'un des radicaux:

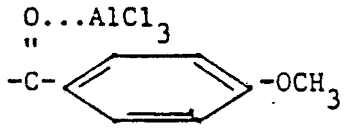

ou

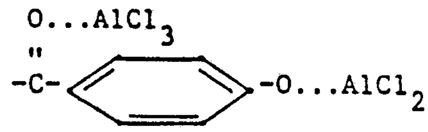

caractérisées en ce qu'elles sont formées au départ d'une ou deux moles de chlorure d'aluminium par mole d'un alkyl-2 (méthoxy-4 benzoyl)-3 benzofuranne de formule générale:

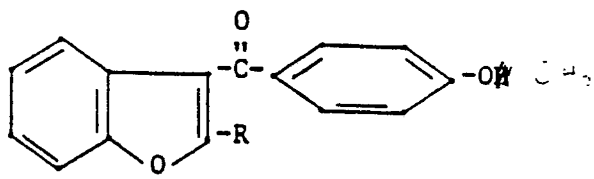

dans laquelle R représente un radical alkyle, et d'un hydrocarbure aromatique.

5. Procédé de préparation de solutions d'un complexe moléculaire correspondant à la formule générale:

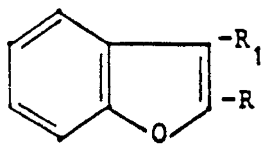

I

dans laquelle R représente un radical alkyle et $R_1$ représente le radical

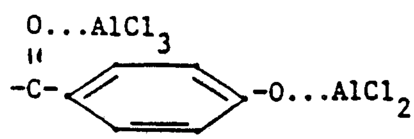

caractérisé en ce que dans un hydrocarbure aromatique au reflux, on coule une solution formée, à température inférieure ou égale à la température ambiante, par un hydrocarbure aromatique et une à deux moles de chlorure d'aluminium par mole d'un alkyl-2 (méthoxy-t benzoyl)-3 benzofuranne.

6. Procédé de préparation d'alkyl-2 (hydroxy-4 benzoyl)-3 benzofuranne de formule générale:

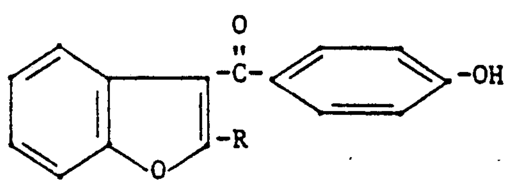

dans laquelle R représente un radical alkyle, selon lequel on fait réagir un alkyl-2 benzofuranne de formule générale:

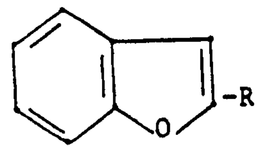

dans laquelle R a la même signification que précédemment, et un halogénure de méthoxy-4 benzoyle, à une température comprise entre -10°C et la température ambiante et en présence de chlorure ferrique comme catalyseur puis on hydrolyse en présence d'un acide fort pour obtenir une solution d'alkyl-2 (méthoxy-4 benzoyl)-3 benzofuranne correspondant, caractérisé en ce qu'on introduit, à température inférieure ou égale à la température ambiante 1 à 2 moles de chlorure d'aluminium par mole d'alkyl-2 (méthoxy-4 benzoyl)-3 benzofuranne puis on coule, dans un hydrocarbure aromatique au reflux, la solution ainsi formée, on hydrolyse en présence d'un acide fort à une température de l'ordre de 75 à 80°C, pour obtenir l'alkyl-2 (hydroxy-4 benzoyl)-3 benzofuranne désiré.

7. Procédé selon l'une quelconque des revendications 1, 2, 3, 5 ou 6, caractérisé en ce que l'hydrocarbure aromatique est le toluène.

**Claims** for the Contracting States: BE, CH, DE, FR, GB; IT, LI, LU, NL, SE

1. Molecular complexes corresponding to the general formula:

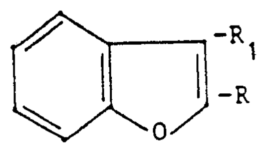

I

in which R represents an alkyl radical and $R_1$ represents one of the radicals:

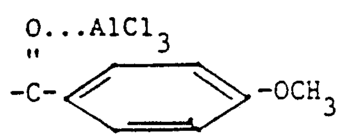

or

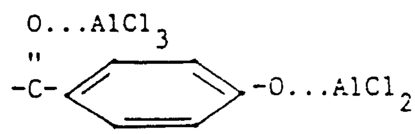

2. Molecular complexes according to claim 1, characterised in that R represents an ethyl group or an n-butyl group.

3. Process for the preparation of molecular complexes according to any one of claims 1 or 2 of formula I, in which $R_1$ represents the radical:

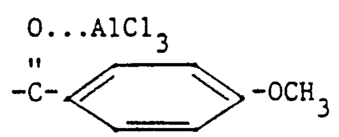

characterised in that aluminium chloride and a 2-alkyl-3-(4-methoxybenzoyl)-benzofuran of the general formula,

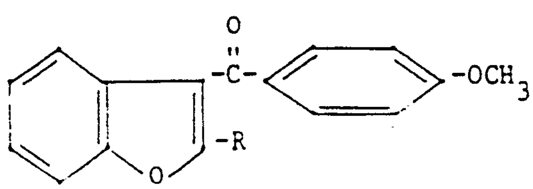

in which R represents an alkyl radical are allowed to react in an aromatic hydrocarbon medium or in a chloroalkane medium at a temperature below or equal to the ambient temperature in a molar ratio of 1 to 2 : 1.

4. Process for the preparation of molecular complexes according to any one of claims 1 or 2 of formula I, in which $R_1$ represents the radical

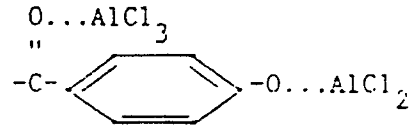

characterised in that a solution containing the molecular complex of formula I according to any one of claims 1 or 2 in which $R_1$ represents the radical

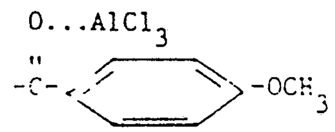

is refluxed, the solution being formed at a temperature below or equal to ambient temperature from an aromatic hydrocarbon and one to two moles of aluminium chloride per mole of 2-alkyl-3-(methoxy benzoyl)-benzofuran of the general formula:

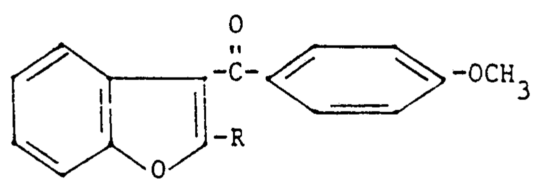

in which R represents an alkyl radical.

5. Process according to claim 4, characterised in that a solution formed at a temperature below or equal to ambient temperature from an aromatic hydrocarbon and 1 to 2 moles of aluminium chloride per mole of 2-alkyl-3-(4-methoxybenzoyl)-benzofuran is poured into an aromatic hydrocarbon under reflux.

6. Solutions containing molecular complexes according to any one of claims 1 or 2, characterised in that they are formed from 1 to 2 moles of aluminium chloride per mole of 2-alkyl-3-(4-methoxybenzoyl)-benzofuran of the general formula

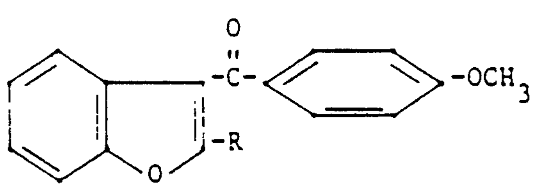

in which R represents an alkyl radical, and from an aromatic hydrocarbon.

7. Process for the preparation of solutions containing molecular complexes according to any one of claims 1 or 2 of formula I, in which $R_1$ represents the radical

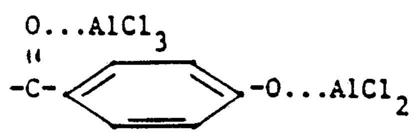

characterised in that a solution formed at a temperature below or equal to ambient temperature by an aromatic hydrocarbon and one to two moles of aluminium chloride per mole of a 2-alkyl-3-(4-methoxybenzoyl)-benzofuran is poured into an aromatic hydrocarbon under reflux.

8. Process for the preparation of 2-alkyl-3-(4-hydroxybenzoyl)-benzofuran of the general formula

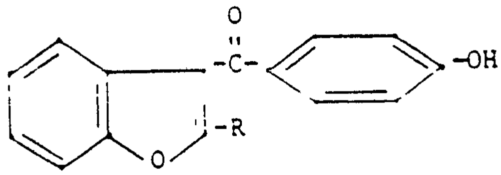

in which R represents an alkyl radical according to which a 2-alkylbenzofuran of the general formula

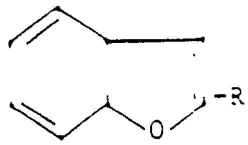

in which R has the same meaning as before, and a 4-methoxy benzoyl halide are allowed to react at a temperature between -10°C and the ambient temperature and in the presence of ferric chloride as a catalyst, followed by hydrolysis in the presence of a strong acid in order to obtain a respective 2-alkyl-3-(4-methoxybenzoyl)-benzofuran solution, characterised in that at a temperature below or equal to the ambient temperature 1 to 2 moles of aluminium chloride per mole 2-alkyl-3-(4-methoxybenzoyl)-benzofuran are introduced, and the solution thus formed is poured into an aromatic hydrocarbon under reflux and then hydrolysed in the presence of a strong acid at a temperature in the region of 75 to 80°C in order to obtain the desired 2-alkyl-3-(4-hydroxybenzoyl)-benzofuran.

9. Process according to any one of claims 3, 4, 5, 7 or 8 characterised in that the aromatic hydrocarbon is toluene.

**Claims** for the Contracting State: AT

1. Process for the preparation of molecular complexes corresponding to the general formula

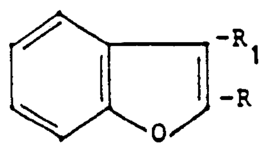

I

in which R represents an alkyl radical and $R_1$ represents the radical

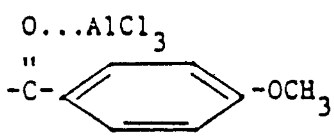

characterised in that aluminium chloride and a 2-alkyl-3-(4-methoxybenzoyl)-benzofuran of general formula

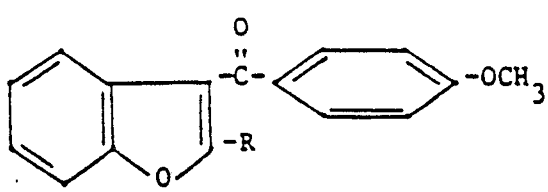

in which R represents an alkyl radical is allowed to react in a molar ratio of 1 to 2 : 1 in an aromatic hydrocarbon medium or a chloroalkane medium at a temperature below or equal to ambient temperature.

2. Process for the preparation of molecular complexes corresponding to the general formula

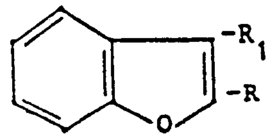

in which R represents an alkyl radical and $R_1$ represents the radical

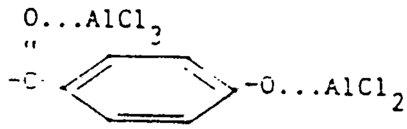

characterised in that a solution containing the molecular complex of formula I in which $R_1$ represents the radical

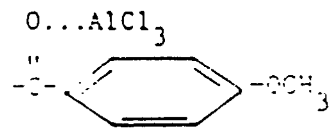

is brought under reflux,

the solution being formed at a temperature below or equal to the ambient temperature from an aromatic hydrocarbon and 1 to 2 moles of aluminium chloride per mole of 2-alkyl-3-(4-ethoxybenzoyl)-benzofuran of the general formula

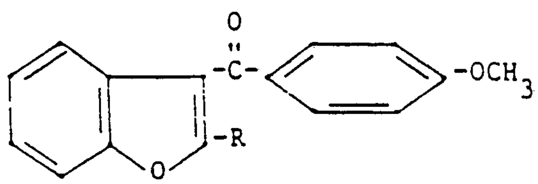

in which R represents an alkyl radical.

3. Process according to claim 2, characterised in that a solution formed at a temperature below or equal to ambient temperature from an aromatic hydrocarbon and one to two moles of aluminium chloride per mole of 2-alkyl-3-(4-methoxybenzoyl)-benzofuran is poured into an aromatic hydrocarbon under reflux.

4. Solutions containing molecular complexes according to the general formula

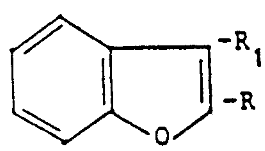

I

in which R represents an alkyl radical and $R_1$ represents one of the radicals

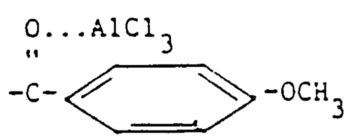

or

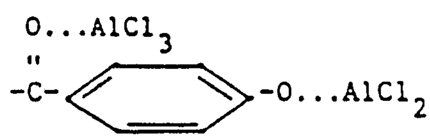

characterised in that they are formed from one or two moles of aluminium chloride per mole of 2-alkyl-3-(4-methoxybenzoyl)-benzofuran of the general formula

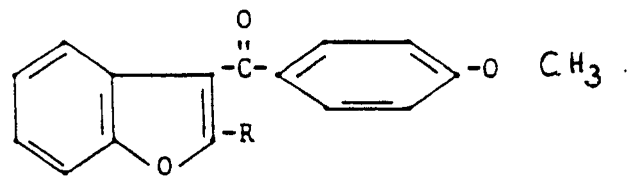

in which R represents an alkyl radical, and from an aromatic hydrocarbon.

5. Process for the preparation of solutions of a molecular complex corresponding to the general formula

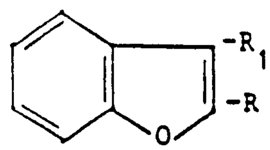

I

in which R represents an alkyl radical and $R_1$ represents the radical

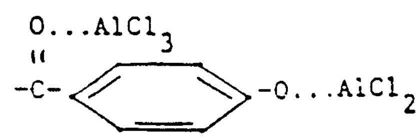

characterised in that a solution formed at a temperature below or equal to the ambient temperature from an aromatic hydrocarbon and one to two moles of aluminium chloride per mole of a 2-alkyl-3-(4-methoxybenzoyl)-benzofuran is poured into an aromatic hydrocarbon under reflux.

6. Process for the preparation of 2-alkyl-3-(4-hydroxybenzoyl)-benzofuran of the general formula

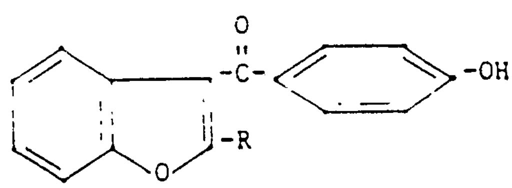

in which R represents an alkyl radical according to which a 2-alkylbenzofuran of the general formula

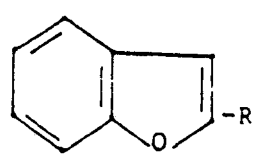

in which R has the same meaning as before, and a 4-methoxy-benzoyl halide are allowed to react at a temperature of between -10°C and ambient temperature in the presence of ferric chloride as a catalyst, followed by hydrolysis in the presence of a strong acid in order to obtain a respective solution of 2-alkyl-3-(methoxybenzoyl)-benzofuran, characterised in that 1 to 2 moles of aluminium chloride per mole of 2-alkyl-3-(4-methoxybenzoyl)-benzofuran are introduced at a temperature below or equal to the ambient temperature and then poured into an aromatic hydrocarbon under reflux, and the solution thus formed is then hydrolysed in the presence of a strong acid at a temperature in the region of 75 to 80°C in order to obtain the desired 2-alkyl-3-(4-hydroxybenzoyl)-benzofuran.

7. Process according to any one of claims 1, 2, 3, 5 or 6, characterised in that the aromatic hydrocarbon is the toluene.

**Patentansprüche** für die Vertragsstaaten: BE, CH, DE, FR, GB, IT, LI, LU, NL, SE

1. Molekulare Komplexe der allgemeinen Formel (I)

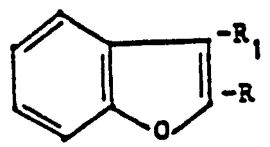

(I),

in der
R Alkyl
und
$R_1$ einer der Reste

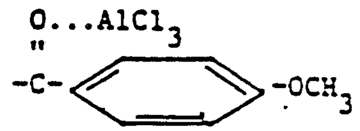

oder

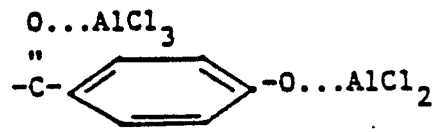

ist.

2. Molekulare Komplexe nach Anspruch 1,
dadurch gekennzeichnet, daß
in Formel (I) R Ethyl oder n-Butyl ist.

3. Verfahren zur Herstellung der molekularen Komplexe nach Anspruch 1 oder 2 der Formel (I), in der $R_1$ der Rest

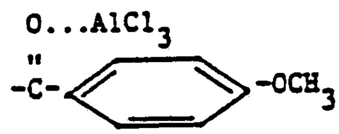

ist,
dadurch gekennzeichnet, daß
Aluminiumchlorid und ein 2-Alkyl-3-(4-methoxybenzoyl)-benzofuran der allgemeinen Formel

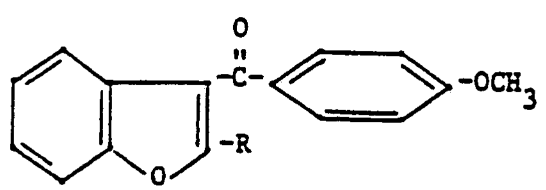

,

in der R Alkyl ist,
in einem molaren Verhältnis von 1 bis 2 : 1 in einem aromatischen Kohlenwasserstoff- oder Chloralkan-Medium bei einer Temperatur unter oder gleich Raumtemperatur umgesetzt werden.

4. Verfahren zur Herstellung der molekularen Komplexe nach Anspruch 1 oder 2 der Formel (I), in der $R_1$ der Rest

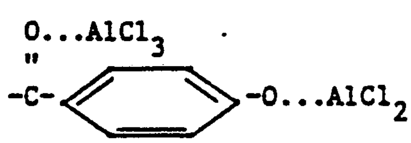

ist,
dadurch gekennzeichnet, daß
eine den molekularen Komplex nach Anspruch 1 oder 2 der Formel (I), in der $R_1$ der Rest der Formel

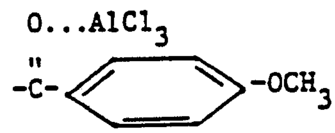

ist, enthaltende Lösung, die bei einer Temperatur unter oder gleich Raumtemperatur aus einem aromatischen Kohlenwasserstoff und 1 bis 2 Mol Aluminiumchlorid je Mol 2-Alkyl-3-(4-methoxybenzoyl)-benzofuran der allgemeinen Formel

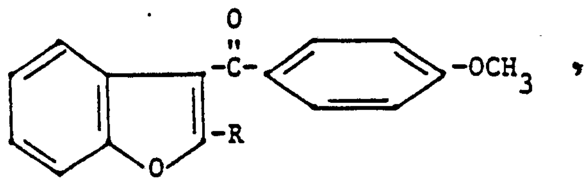

in der R Alkyl ist,
gebildet worden ist, unter Rückfluß erhitzt wird.

5. Verfahren nach Anspruch 4, dadurch gekennzeichnet, daß in einen unter Rückfluß siedenden aromatischen Kohlenwasserstoff eine bei einer Temperatur unter oder gleich Raumtemperatur aus einem aromatischen Kohlenwasserstoff und 1 bis 2 Mol Aluminiumchlorid je Mol 2-Alkyl-3-(4-methoxybenzoyl)-benzofuran gebildete Lösung eingegossen wird.

6. Die molekulare Komplexe nach Anspruch 1 oder 2 enthaltende Lösungen,
dadurch gekennzeichnet, daß
sie aus 1 bis 2 Mol Aluminiumchlorid je Mol 2-Alkyl-3-(4-methoxybenzoyl)-benzofuran der allgemeinen Formel

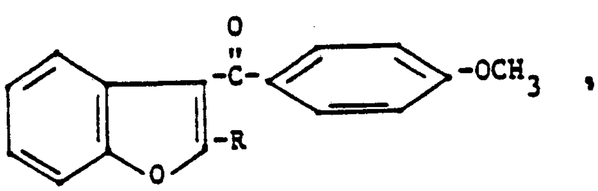

in der R Alkyl ist,
und einem aromatischen Kohlenwasserstoff gebildet werden.

7. Verfahren zur Herstellung der Lösungen, die die molekularen Komplexe nach Anspruch 1 oder 2 der Formel (I) enthalten, in der $R_1$ der Rest

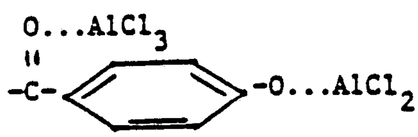

ist,
dadurch gekennzeichnet, daß in einen unter Rückfluß siedenden aromatischen Kohlenwasserstoff eine bei einer Temperatur unter oder gleich Raumtemperatur gebildete Lösung aus einem aromatischen Kohlenwasserstoff und 1 bis 2 Mol Aluminiumchlorid je Mol 2-Alkyl-3-(4-methoxybenzoyl)-benzofuran eingegossen wird.

8. Verfahren zur Herstellung von 2-Alkyl-3-(4-hydroxybenzoyl)-benzofuran der allgemeinen Formel

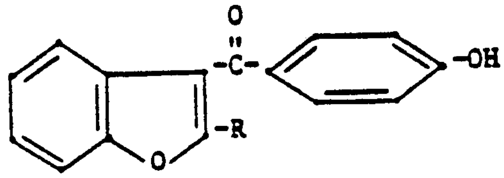

,

in der R Alkyl ist,

durch Umsetzung eines 2-Alkylbenzofurans der allgemeinen Formel

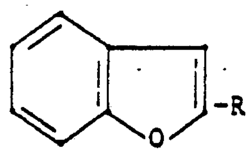

,

in der R die oben angegebene Bedeutung hat,

und eines 4-Methoxybenzoylhalogenids bei einer Temperatur von -10°C bis Raumtemperatur in Gegenwart von Eisen(III)-chlorid als Katalysator und Hydrolyse in Gegenwart einer starken Säure zu einer Lösung des entsprechenden 2-Alkyl-3-(4-methoxybenzoyl)-benzofurans,

dadurch gekennzeichnet, daß

bei einer Temperatur unter oder gleich Raumtemperatur 1 bis 2 Mol Aluminiumchlorid je Mol 2-Alkyl-3-(4-methoxybenzoyl)-benzofuran eingebracht werden, die so gebildete Lösung in einen unter Rückfluß siedenden aromatischen Kohlenwasserstoff eingegossen wird und in Gegenwart einer starken Säure bei einer Temperatur von 75 bis 80°C zu dem gewünschten 2-Alkyl-3-(4-hydroxybenzoyl)-benzofuran hydrolysiert wird.

9. Verfahren nach einem der Ansprüche 3 bis 5, 7 oder 8, dadurch gekennzeichnet, daß als aromatischer Kohlenwasserstoff Toluol verwendet wird.

**Patentansprüche** für den Vertragsstaat: AT

1. Verfahren zur Herstellung von molekularen Komplexen der allgemeinen Formel (I)

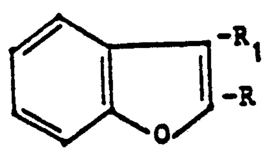

(I),

in der

R Alkyl und

$R_1$ der Rest

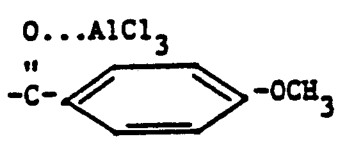

ist,

dadurch gekennzeichnet, daß

Aluminiumchlorid und ein 2-Alkyl-3-(4-methoxybenzoyl)-benzofuran der allgemeinen Formel

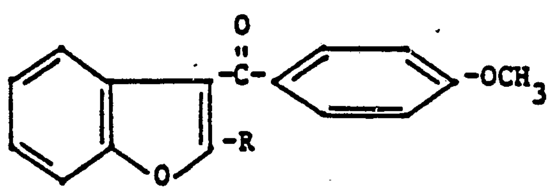

in der R Alkyl ist,

in einem molaren Verhältnis von 1 bis 2 : 1 in einem aromatischen Kohlenwasserstoff- oder Chloralkan-Medium bei einer Temperatur unter oder gleich Raumtemperatur umgesetzt werden.

2. Verfahren zur Herstellung von molekularen Komplexen der allgemeinen Formel

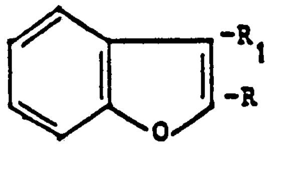

,

in der R Alkyl
und
$R_1$ der Rest

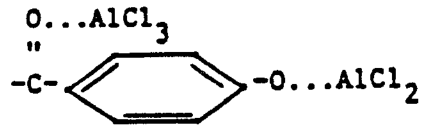

ist,
<u>dadurch gekennzeichnet</u>, daß
eine den molekularen Komplex der Formel (I), in der $R_1$ der Rest der Formel

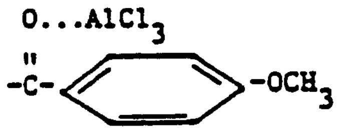

ist, enthaltende Lösung, die bei einer Temperatur unter oder gleich Raumtemperatur aus einem aromatischen Kohlenwasserstoff und 1 bis 2 Mol Aluminiumchlorid je Mol 2-Alkyl-3-(4-methoxybenzoyl)-benzofuran der allgemeinen Formel

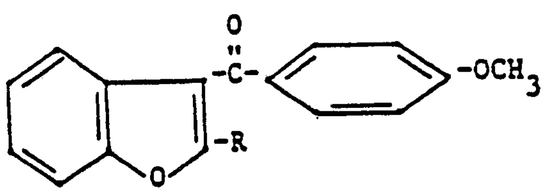

,

in der R Alkyl ist,
erhalten worden ist, unter Rückfluß erhitzt wird.

3. Verfahren nach Anspruch 2, dadurch gekennzeichnet, daß in einen unter Rückfluß siedenden Kohlenwasserstoff eine bei einer Temperatur unter oder gleich Raumtemperatur gebildete Lösung aus einem aromatischen Kohlenwasserstoff und 1 bis 2 Mol Aluminiumchlorid je Mol 2-Alkyl-3-(4-methoxybenzoyl)-benzofuran gebildete Lösung eingegossen wird.

4. Lösungen, die die molekularen Komplexe der allgemeinen Formel (I) enthalten,

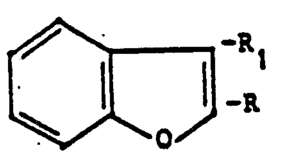

(I),

in der R Alkyl und
$R_1$ einer der Reste

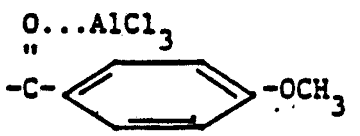

oder

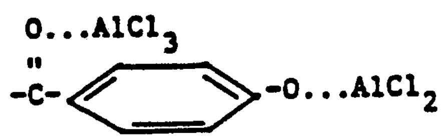

ist,
<u>dadurch gekennzeichnet</u>, daß
sie aus 1 bis 2 Mol Aluminiumchlorid je Mol 2-Alkyl-3-(4-methoxybenzoyl)-benzofuran der allgemeinen Formel

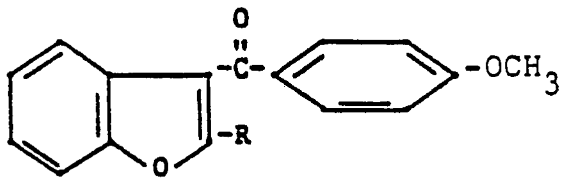
,

in der R Alkyl ist,
und einem aromatischen Kohlenwasserstoff bestehen.

5. Verfahren zur Herstellung der Lösungen eines molekularen Komplexes der allgemeinen Formel (I)

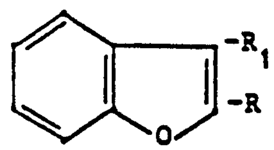
(I),

in der R Alkyl
und
$R_1$ der Rest

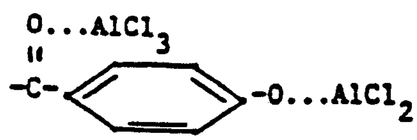

ist,
dadurch gekennzeichnet, daß
in einen unter Rückfluß siedenden aromatischen Kohlenwasserstoff eine bei einer Temperatur unter oder gleich Raumtemperatur gebildete Lösung aus einem aromatischen Kohlenwasserstoff und 1 bis 2 Mol Aluminiumchlorid je Mol 2-Alkyl-3-(4-methoxybenzoyl)-benzofuran gebildete Lösung eingegossen wird.

6. Verfahren zur Herstellung von 2-Alkyl-3-(4-hydroxybenzoyl)-benzofuran der allgemeinen Formel

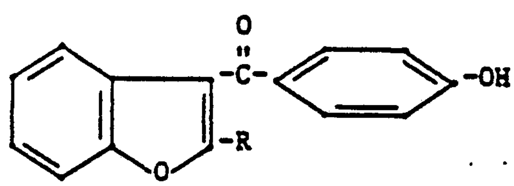
,

in der R Alkyl ist,
durch Umsetzung eines 2-Alkylbenzofurans der allgemeinen Formel

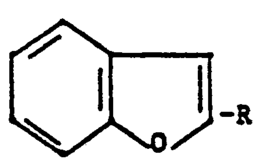
,

in der R die oben angegebene Bedeutung hat,
und eines 4-Methoxybenzoylhalogenids bei einer Temperatur von -10 °C bis Raumtemperatur in Gegenwart von Eisen(III)-chlorid als Katalysator und Hydrolyse in Gegenwart einer starken Säure zu einer Lösung des entsprechenden 2-Alkyl-3-(4-methoxybenzoyl)-benzofurans,
dadurch gekennzeichnet, daß
1 bis 2 Mol Aluminiumchlorid je Mol 2-Alkyl-3-(4-methoxybenzoyl)-benzofuran bei einer Temperatur unter oder gleich Raumtemperatur eingebracht werden und in einen unter Rückfluß siedenden aromatischen Kohlenwasserstoff die so gebildete Lösung eingegossen wird und in Gegenwart einer starken Säure bei einer Temperatur von 75 bis 80° C zu dem gewünschten 2-Alkyl-3-(4-hydroxybenzoyl)-benzofuran hydrolysiert wird.

7. Verfahren nach einem der Ansprüche 1 bis 3, 5 oder 6, dadurch gekennzeichnet, daß als aromatischer Kohlenwasserstoff Toluol verwendet wird.